# EUROPEAN PATENT APPLICATION

(11) **EP 4 063 782 A1**
(43) Date of publication of application: **28.09.2022**
(21) Application number: 22173874.3
(22) Date of filing: 05.10.2016
(51) Int. Cl.: G01B 11/02, A43D 1/02

(54) **FOOT SHAPE ACQUISITION USING DEPTH SENSOR AND PRESSURE PLATE TECHNOLOGY**

(62) Divisional of application: 16788806.4
(71) Applicant: SafeSize Holding B.V., 1314 SK Almere (NL); UCS kupcu prilagojeni proizvodi, d.o.o., 1000 Ljubljana (SI)
(72) Inventor: LAHAJNAR, Leon, 5282 Cerkno (SI); OMRCEN, Damir, 1000 Ljubljana (SI); KOLSEK, Tomaz, 1000 Ljubljana (SI); STAVRAKIS, Angelos, 7442 DR Nijverdal (NL)
(74) Representative: V.O.

(57) **Abstract**

An apparatus characterized by using at least three depth sensors to acquire shape of one or two feet, wherein at least two depth sensors are positioned above the front part of the foot whereas at least one depth sensor is positioned at the back of the foot facing the heel.

## Description

### FIELD

The present disclosure generally relates to foot measuring devices, IPC A61B5/1074 Foot measuring devices, A43D1/025 Foot or last measuring devices.

### BACKGROUND

This section provides background information, which is not necessarily prior art.

The traditional footwear industry and sales relies on the assumption that a shoe of particular length should fit a foot of a corresponding length. Therefore, shoes are traditionally manufactured in sizes with a particular step (6.67mm in EU sizing, 8.4mm for UK sizing, etc.).

It is also assumed that tolerances within manufacturing should not result in a considerable shoe length dispersion within a selected size. On the other hand, people with particular foot length (measured in mm) are assumed to select a particular size. However, it can be proved that both above assumptions are false to a large extent. Invalidity of these assumptions may have severe negative consequences in some businesses, such as internet shoe sales, where the end consumer does not have a chance of trying the individual sizes prior to purchase.

For proper fit, the accurate measurement of both shoes and feet is necessary. It has been proved, that although important, the length is not the only relevant characteristics of shoe and the foot. For a foot measurement, the process has to be made fast, reliable, accurate, easy to use and cheap. There are number of devices for foot measurement already available, based on various working principles. For example, these principles are:

Manual driven mechanical devices, originating from "Brannock" device, which usually have a static heel barrier, and a manual driven slider, which slides along measurement tape. The slider is pushed with a low force against human foot. Variants of this device can measure also width of the foot and sometimes the ball length.

Automated mechanical device, where pushing of sliders is implemented by means of electrical or pneumatic or other energy source, and stopping of a slider is implemented either by sensing the pressure against the foot or optically.

Laser based measurement devices, which use various laser projectors, which emit a particular light pattern, typically a line, and a camera, which records the emitted light. By knowing the position of both projector and a camera, it is possible to reconstruct the depth information and 3D coordinates of laser-illuminated area.

Ordinary light in combination with highly patterned socks. The images of deformed sock patterns can be analyzed to arrive at depth information.

In past years' new affordable optical sensors have been developed, which contain both a light pattern emitter and the camera on the same printed circuit board (PCB). The emitter emits several different light patterns with a high frequency, whereas the camera captures these patterns. Vendor supplied software is able to reconstruct the "depth" of each pixel, hence the name "depth sensor" or "depth camera". Usually the applied light frequency is from infra-red spectrum, and the detection distance range can vary from 18cm to several meters. Examples of these sensors are Intel-RealSense, Occiptal-Structure, Microsoft-Kinect, Asus Xtion, etc.

Pressure plates have been in use for several years by podiatrists, clinical examination, sport shoe sales and biometric researchers. They can provide information on walking irregularities, pronation and supination, etc. Vendors supply pressure plates with analysis software with various utility analysis, such as calculation of pressure line, maximum value tracking, pressure versus time, etc.

Various applications require reliable and accurate foot scanners. For example, podiatrists measure humans feet to design and eventually manufacture custom made insoles, which fit a particular individual. Another example is application in running sport, where it is important that a particular shoe type is properly selected with respect to human feet shape, especially with respect to arch height. And an example of usage is in the shoe retail, where it is important to select a proper size of the shoe with respect to particular foot of the individual.

Every particular application may have different expectations from a foot scanner. It is assumed to be reliable, accurate, and preferably cheap. However, these requirements may not all be fulfilled to the very high level.

The present invention claims a solution, which can offer reliable, accurate and low cost foot scanner for various fields of application, not limiting themselves to the ones presented above.

### BRIEF DESCRIPTION OF THE DRAWINGS

The drawings described herein are for illustrative purposes only of selected embodiments and are not intended to limit the scope of the present disclosure.
FIG.1 is showing an apparatus for measurement of feet shape with single depth sensor with the following elements: Feet (1), Depth Sensor (2), Heel Barrier (3);
FIG.2 is showing an apparatus for measurement of feet shape with single depth sensor in combination with pressure plate with the following elements: Feet (1), Depth Sensor (2), Heel Barrier (3), Pressure Plate (4);
FIG.3 is showing an apparatus for measurement of feet shape with two depth sensors with the following elements: Feet (1), Depth Sensors (2), Heel Barrier (3);
FIG.4 is showing an apparatus for measurement of feet shape with two depth sensors in combination with pressure plate with the following elements: Feet (1), Depth Sensors (2), Heel Barrier (3), Pressure Plate (4);
FIG.5 is showing an apparatus for measurement of feet shape with three depth sensors with the following elements: Feet (1), Depth Sensors (2);
FIG.6 is showing an apparatus for measurement of feet shape with three depth sensors in combination with pressure plate with the following elements: Feet (1), Depth Sensors (2), Pressure Plate (4);
FIG.7 is showing an apparatus for measurement of feet shape with four depth sensors with the following elements: Feet (1), Depth Sensors (2);
FIG.8 is showing an apparatus for measurement of feet shape with four depth sensors in combination with pressure plate with the following elements: Feet (1), Depth Sensors (2), Pressure Plate (4); and
FIG.9 is showing a typical foot measurement apparatus together with processing and display devices with the following elements: Feet (1), Depth Sensors (2), Pressure Plate (4), Display Device (5), Processing Device (6), Mobile Device (7).

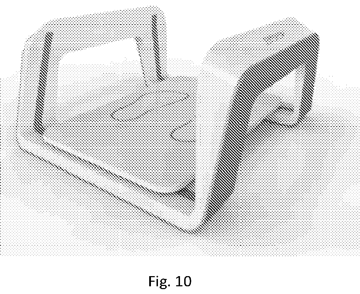

FIG.10 is showing the design of the commercial execution of the apparatus.

### DETAILED DESCRIPTION

Preferred embodiments of the invention are disclosed here, however, these embodiments are merely examples of the invention, which may be embodied in various forms.

The design and functions disclosed should not be interpreted as limiting, but as a representative to employ invention in any appropriately detailed system.

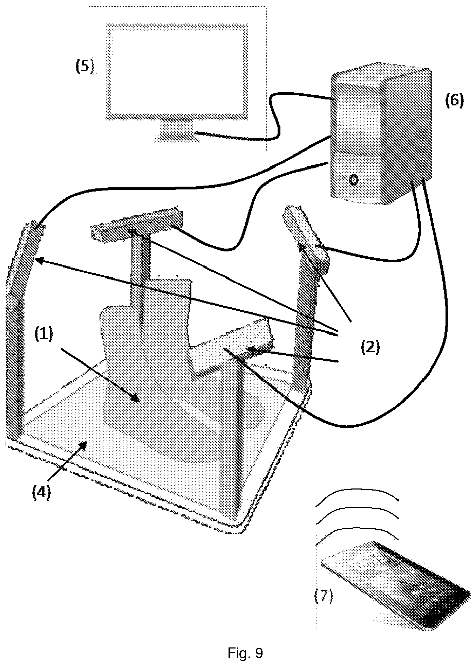

Fig. 9 is showing a typical foot measurement apparatus together with processing and display devices. Foot measurement apparatus consists of: a) mandatory depth sensor, b) optional pressure plate sensor, c) mandatory processing and control device, such as personal computer, d) optional display device, such as computer monitor or TV or a mobile tablet.

The process begins by end consumer taking off his shoes, rolling up his pants and stepping inside measurement area. Process continues by triggering the measurement on the processing and controlling device (PC). This starts measurement with depth sensors.

Depth sensors contain infra-red light pattern emitter, which drops several different light patterns with a high frequency onto the foot surface.

The emitted light is capture by a camera on the depth sensor. This results in a raster image of n x M pixels. The software provided by depth sensor vendor is capable of calculating the depth of each pixel by using triangulation method.

Each pixel with depth coordinate can be considered as a point in space. Points coming from a single sensor are called point clouds. Since sensors may pick up some noise, the point clouds have to be filtered by special software to eliminate as much noise as possible. Potential multiple point clouds coming from potential multiple sensors are combined to cover as much of foot surface as possible. To reduce the time for scanning, it is best to cover as much as possible of both feet surfaces in a single measurement. To be able to combine point clouds from multiple depth sensors, a transformation matrix has to be computed for each sensor. This can be achieved in multiple ways, for example by putting a rigid body of simple shape, which has a known geometry.

A point cloud may already suffice for certain applications, for example to extract some simple linear dimensions, such as length and width of the foot. If the surface coverage is good enough, it may be possible to extract partial or full silhouettes, for example side silhouette and top silhouette.

Point cloud may further be processed to create a triangulated surface. The advantages of such data form are easy and fast display of surface, ease of extraction of girths, less needed storage, etc.

The foot surface and extracted relevant dimensions are shown via graphical user interface to both end consumer or sales assistant. Data may also be stored in a central database for further usage.

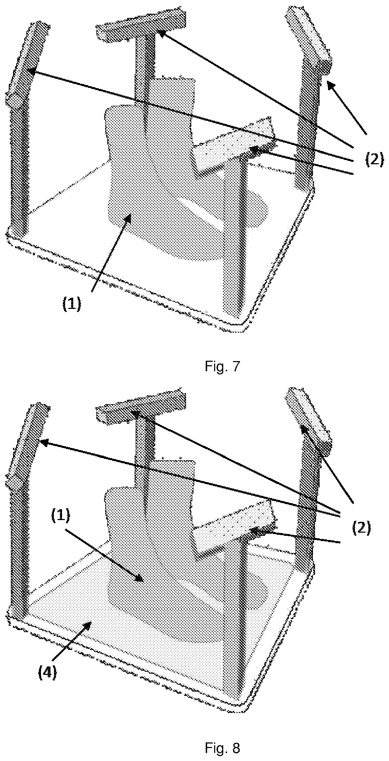

A complementary data can be received from a pressure plate sensor, which acquires pressure distribution between feet and the floor. A measurement apparatus can be formed, for example as depicted in Fig. 8, where end consumer can walk through apparatus, and pressure plate sensor dynamically records the pressure. By analyzing such data, it is possible to detect particular types of gait, pronation/supination, potential problems, etc.

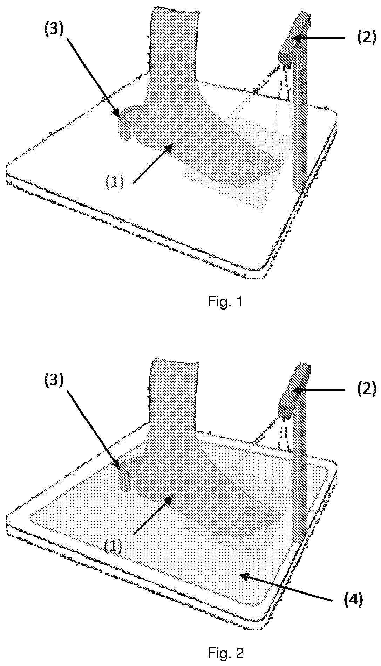

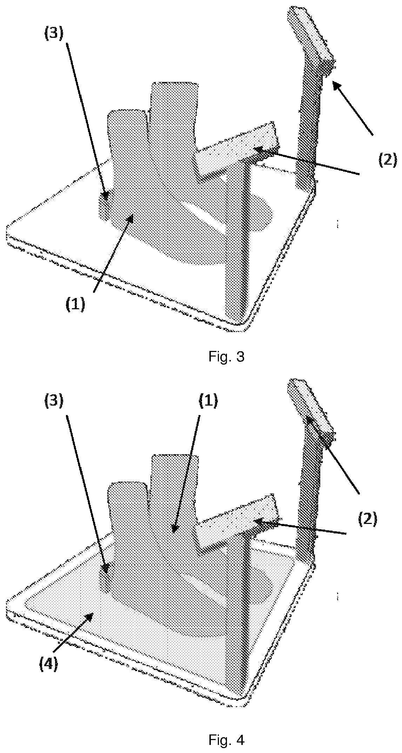

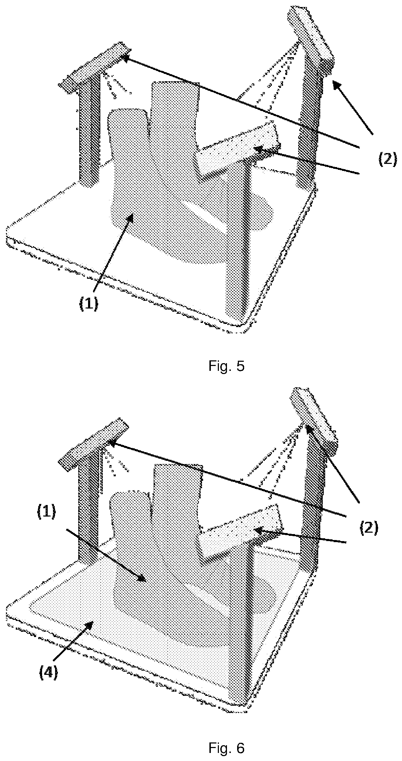

Figures 1-8 are showing other possible implementations of the invention. The simplest implementation uses only one depth camera (Figure 1). By adding additional elements to the measurement apparatus we get more complex solutions (Figures 2-8), which, on the other hand, result in higher reliability and accuracy. The drawback of adding the elements is the increase in a cost of a device.

### CITATIONS

US5128880A Foot measurement and footwear sizing system.
US5361133A Method and apparatus for analyzing feet.
US6289107B1 Apparatus and method of measuring human extremities using peripheral illumination techniques.
US6549639B1 Body part imaging system.
US3192627A Foot measuring and pedograph revealing machine.
US6834437B1 Foot measurement system.
US6160264A System for plotting a tri-dimensional shape, notably a plantar arch, and method of operating the system for producing an orthopaedic shoe or sole.
US8117922B2 Footcare product dispensing kiosk.
US20070253004A1 Foot Measuring Device.
US2942344A Foot measuring device.
US7421789B1 Systems and methods for footwear related measurement and adjustment. EP0014022A1 Foot-size measuring apparatus.
US20030164954A1 Device for optoelectronically determining the length and/or the width of a body situated on a support.
US2200223A Foot measuring apparatus.
EP0760622B1 Digitised sensing process and arrangement for the three-dimensional shape in space of bodies or body parts.
US4164815A Device for measuring a human foot.
US5689446A Foot contour digitizer.
US3018554A Foot measuring device.
EP1418398A1 Shape measuring device.
US7516555B2 Systems and methods for footwear related measurement and adjustment. US20100286951A1 Foot measuring device.
US20100296726A1 High-resolution optical detection of the three-dimensional shape of bodies.
WO2004037085A1 APPARATUS FOR DETERMINING SIZE AND SHAPE OF A FOOT. US20140182152A1 Footgauge.
DE102011007678A1 Measurement system for determining biometric data of human foot of children during shoe purchase, aligns human foot on boundary surface with respect to measurement coordinate system.
EP2954798A1 Foot scanner.
DE102010019234A1 Foot measuring device for e.g. human, for assisting individual during manufacture of shoe, has evaluation unit determining three-dimensional shape of part of foot from output signals of sensors i.e. ultrasonic sensors
DE102013001897A1 Method for determining biometric data of limb e.g. human foot, involves providing image acquisition generated by orientation sensor together with image analysis process data for determining measurement data and biometric data
US3931680A Foot measuring machines
US20030137510A1 Method and assembly for the photogrammetric detection of the 3-d shape of an object

## Claims

1. An apparatus **characterized by** using at least three depth sensors to acquire shape of one or two feet, wherein at least two depth sensors are positioned above the front part of the foot whereas at least one depth sensor is positioned at the back of the foot facing the heel.

2. An apparatus according to claim 1, **characterized by** using four depth sensors to acquire shape of one or two feet, wherein two depth sensors are positioned in the front of the foot and two depth sensors are positioned at the back of the foot.

3. An apparatus according to claim 1 or 2, **characterized by** five or more depth sensors to acquire shape of one or two feet.

4. The apparatus according to any one of claims 1 - 3, wherein an additional pressure plate is used, the apparatus being capable of acquisition of foot shape, as well as the static pressure distribution over human sole.

5. An apparatus according to any one of claims 1 - 4, wherein the apparatus is free of any heel barrier.

6. An apparatus according to any one of claims 1 - 5, wherein the apparatus further comprises a display device, a processing device and a mobile device.

7. An apparatus according to any one of the previous claims, wherein depth sensors contain infra-red light pattern emitters.

8. A procedure where information yielded from multiple depth sensors is transformed into a unified coordinate system and a transformation is calculated for each sensor, wherein the data comes from multiple depth sensors of an apparatus according to any one of claims 1 - 7, providing point clouds coming from multiple sources which are put into single coordinate system.

9. A procedure according to claim 8, using a rigid body with even surfaces and known angles between surfaces, an example of such body being a brick or a cube, to determine sensor locations in space, and thus put point clouds from different sources into a single coordinate system.

10. A procedure for filtering of data from depth sensors of an apparatus according to any one of claims 1 - 7, wherein point clouds from different depth sensors are put into in a single coordinate system, **characterized by** application of two steps:
(10.1) application of filtering to each point cloud,
(10.2) application of filtering to combined point clouds.

11. A procedure according to claim 10, **characterized by**:
(11.1) extraction of linear dimensions directly by application of bounding box method, such as extracting foot length, foot width, etc.,
(11.2) extracting typical contours, such as side silhouette and top silhouette,
(11.3) apply point cloud triangulation algorithm, which results in an open or closed triangulated surface. The triangulated surface allows easier extraction of important foot characteristics, such as girths at arbitrary location.

12. Method according to any one of claims 8 - 11, wherein an image of at least one foot is obtained, based on the point clouds of each depth sensor, wherein for each pixel of said image the depth of the pixel is calculated by using a triangulation method.

13. Method according to any one of claims 8 - 12, wherein a foot surface and relevant dimensions are shown via a graphical user interface, wherein data obtained is preferably stored in a central database for further usage.
